# EUROPEAN PATENT SPECIFICATION

(11) **EP 1 599 137 B1**
(45) Date of publication and mention of the grant of the patent: **02.09.2009**
(21) Application number: 04710090.4
(22) Date of filing: 11.02.2004
(51) Int. Cl.: A61B 8/12, A61B 6/00

(54) **INTRAVASCULAR IMAGING**
INTRAVASKULÄRE BILDDARSTELLUNG
IMAGERIE INTRAVASCULAIRE

(30) Priority: 25.02.2003 EP 03100446
(43) Date of publication of application: 30.11.2005
(73) Proprietor: Philips Intellectual Property & Standards GmbH, 20099 Hamburg (DE); Koninklijke Philips Electronics N.V., 5621 BA Eindhoven (NL)
(72) Inventor: BORGERT, J., Philips Intell. Pty & Standards GmbH, 52066 Aachen (DE); SABCZYNSKI, J, Philips Intell Pty & Standards GmbH, 52066 Aachen (DE)
(74) Representative: Damen, Daniel Martijn
(86) International application number: PCT/IB2004/000351
(87) International publication number: WO 2004/075756

(56) References cited:
- US-A- 5 797 849
- US-A1- 2002 115 931

## Description

The disclosure relates to a method of interventional angiography, in which intravascular image signals are recorded by means of a first image recording device inserted into a blood vessel of a patient, the intravascular position of which image recording device is determined during the recording of the images, where the intravascular image signals are visualized by means of a display unit taking account of the intravascular position of the first image recording device.

The invention relates to a system for interventional angiography for carrying out the abovementioned method, and also to a computer program for this system.

In order to be able to place a stent in an optimal and risk-free manner during an angiographic examination, for example during a left ventricular catheter examination, it is important to know the state of the so-called vulnerable plaque. For this, the physician makes use of suitable imaging methods which allow him to have a look at the condition of the inner walls of the vessels.

A system for carrying out such an imaging method is known for example from WO 02/064011 A2. In said system, a large number of two-dimensional images of the vessel under examination are recorded by means of the image recording device that has been inserted into the vessel, and said two-dimensional images are combined by means of a suitable software application to form a three-dimensional image. In this case, the position and orientation of the image recording device are determined by means of a locating device and taken into account in the calculation of a volume image. The movement of the patient during the intervention is also taken into account.

A disadvantage of this imaging system is that on account of the complex visualization of the volume rendering large amounts of data have to be converted, and this entails a high degree of computational power and hence considerable costs. It is also disadvantageous that the information contained in the three-dimensional volume images can be interpreted by the physician only with difficulty. It is generally necessary for the physician to interactively influence the image representation (viewing angle, rotation, enlargement, section, etc.), and this takes a lot of time and may easily lead to incorrect interpretations of the anatomical structures represented.

An intravascular imaging method is also known in which planar projection images of the blood vessels, known as angiograms, are recorded by means of an X-ray device, usually a C-arm X-ray device. Usually, two angiograms are recorded and displayed synoptically - one real-time angiogram and one reference angiogram. The reference angiogram is a static image which is recorded under the effect of a sprayed contrast medium. The blood vessels can easily be identified therein in a manner similar to a road map. Since contrast media are harmful to the patient on account of their toxic composition, the patient is only exposed to such contrast media for a short time, for the duration of the reference recording.

If, for example, a stent is to be placed, the actual treatment begins after this purely diagnostic step - following breakdown of the contrast medium. In parallel with the X-ray irradiation, an image recording device is inserted into the patient's blood vessel that is to be examined, by means of a catheter. This image recording device is usually based on ultrasound technology and supplies image signals of the vessel inner wall. A two-dimensional representation of the vessel inner wall is calculated from the image signals recorded, and this is displayed on a separate display. The real-time angiogram is recorded at the same time as the catheter examination. In said real-time angiogram the blood vessels can rarely be seen, although the current position of the catheter tip in the body can be seen clearly. For this purpose, radio-opaque markings for example may be applied to the catheter.

The intravascular image signals represented on the separate display provide the physician with additional information about the condition of the plaque in the region of any narrowings of the vessel. By visually comparing the two angiograms, the physician can discover the anatomical location where the intravascular images were taken.

One disadvantage of this method is that it is confusing and taxing for the physician. In order to examine the inner wall of a specific vessel, he must look at three representations in parallel and combine them in his mind.

A device according to the preamble of claim 1 is known from US 2002/0 115 931.

It is therefore an object of the present disclosure to provide a method of interventional angiography in which the aforementioned disadvantages are avoided. In particular, the intention is to provide a method by means of which the recorded image information is processed and displayed in a manner that is clear and convenient for the physician.

This object is achieved by an imaging method of the type mentioned above in which for visualization purposes a two-dimensional representation of the intravascular image signals is superposed on a representation of a two-dimensional projection image of the blood vessel that is generated by means of a second image recording device, in accordance with the intravascular position of the first image recording device.

By virtue of the limitation to a planar representation of the three-dimensional intravascular image signals, in the method according to the invention large amounts of data and complex calculations during the visualization process are avoided. At the same time, the images can be displayed clearly on conventional output devices, for example on simple video monitors. The visual perception of the spatial vessel structures recorded by means of the first image recording device is particularly simple and intuitive for the physician by virtue of the method according to the invention.

A further advantage is that the intravascular image signals are displayed on a display unit together with the planar projection images, rather than on a separate monitor. The physician therefore does not need to take his eye off this display unit.

The intravascular position of the first image recording device may be located in two ways. Firstly, the position can be determined on the basis of the mapping of the first image recording device within the two-dimensional projection image. For this, use is expediently made of an image processing means, which recognizes the shadows of the catheter tip or corresponding markings in the real-time angiogram. Suitable image recognition software has been known for a long time.

The second way of determining the intravascular position of the first image recording device consists in providing a locating device specifically for this purpose. Such devices are being increasingly used in current medical technology and operate in accordance with electromagnetic or optical principles.

In order to obtain a clear, planar representation of the vessel inner wall, it is particularly useful to generate, from the intravascular image signals, a developed view in the image plane of the projection image of the blood vessel. Such a developed view makes it possible to easily see the condition of the pathological plaque deposits so that when placing a stent the risk of undesired thrombus formation can be assessed. As an alternative, there is the possibility for the two-dimensional representation of the intravascular image signals to be for example a representation of a section of the volume rendering recorded by means of the first image recording device, wherein it is useful if the image plane of this representation, that is to say the sectional plane, can be interactively predefined and manipulated by the physician.

Moreover, the two-dimensional representation of the intravascular image signals may be generated by projecting the volume rendering, recorded by means of the first image recording device, into the image plane of the two-dimensional projection image, for example by means of so-called maximum intensity projection (MIP).

The two-dimensional representation of the wall of the blood vessel is expediently generated from a number of slice images recorded by means of the first image recording device and oriented perpendicular to the longitudinal extent of the blood vessel. According to one useful further development of the method according to the invention, the two-dimensional representation of the intravascular image signals is supplemented successively according to the movement of the first image recording device within the blood vessel. As a result, the physician has the option of being able to inspect at any time all vessel sections that have been passed by the first image recording device during the intervention.

A suitable system for interventional angiography, for carrying out the imaging method, comprises a first image recording device for insertion into a blood vessel of the patient, means for determining the intravascular position of the first image recording device, a display unit for visualizing the intravascular image signals recorded by means of the first image recording device, and furthermore of a second image recording device which generates a two-dimensional projection image of the blood vessel, and of image processing means which superpose on the two-dimensional projection image a planar representation of the intravascular image signals in accordance with the intravascular position of the first image recording device.

As an alternative, an intravascular ultrasound device or an optical coherence tomography device are suitable as first image recording device. A C-arm X-ray device can be used as the second image recording device.

A system according to the invention is defined is claim 1.

The image processing means of the system according to the invention can be embodied by a computer on which a suitable program is run. Such a computer program reads the rendering from the first image recording device inserted into a blood vessel of the patient, the projection rendering from the second image recording device and also the intravascular position data from the first image recording device, and calculates therefrom a two-dimensional image by superposing on a representation of the projection rendering a two-dimensional representation of the intravascular rendering in accordance with the intravascular position data from the first image recording device. The advantage of such a computer program is, inter alia, that it can easily be used to retrofit existing systems to the level of the invention. The required computational capability is usually already present.

The computer program generates a particularly clear two-dimensional representation of the intravascular rendering if it calculates, from the intravascular rendering, a two-dimensional representation of the wall of the blood vessel taken in the image plane of the two-dimensional image. A computer program according to the invention is defined in claim 5.

The invention will be further described with reference to examples of embodiments shown in the drawings to which, however, the invention is not restricted.

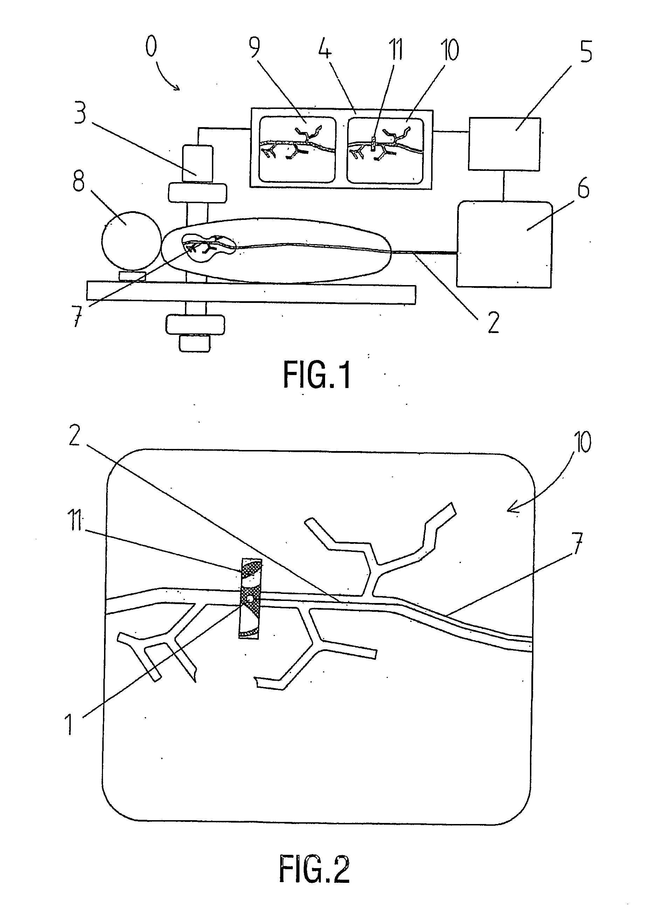

Fig. 1 shows the schematic design of the system with a patient.
Fig. 2 shows an enlargement of a projection image with superposed two-dimensional representation of the intravascular image signal according to the invention.

The system 0 used according to the invention for interventional angiography comprises a first image recording device 1 in the form of an intravascular ultrasound signal converter at the tip of a catheter 2, a second image recording device 3 in the form of a C-arm X-ray device, a display unit 4, an image processing means 5 and an integrated catheter guidance and locating device 6, which operates either optically or electromagnetically. The diagnostic imaging system 0 is used to inspect a blood vessel 7 of a patient 8.

The image recording devices 1, 3, the visualization of the image signals on the display unit 5 and the integrated catheter guidance and locating device 6 have been known from the prior art for a long time and are therefore not described in any more detail in the present text.

In an imaging procedure, an X-ray contrast medium is sprayed onto the patient 8. At the same time, an angiogram, that is to say a planar projection image of the vessel that is to be examined, is recorded by means of the second image recording device 3, a C-arm X-ray device. This is displayed on the display unit 4 as reference image 9. The actual treatment, for example the placing of a stent, begins following breakdown of the contrast medium. For this, a catheter 2 is inserted into the blood vessel 7 of the patient 8, at the tip of which catheter there is the first image recording device 1, an intravascular ultrasound signal converter.

The catheter 2 is guided into the blood vessel 7 to be examined. The intravascular position of the catheter 2 at any given instant is continually determined by the integrated catheter guidance and locating device 6.

During the examination, a real-time image 10 of the blood vessel 7 is recorded by means of the second image recording device 3 and likewise displayed on the display unit 4. The tip of the catheter 2 can be seen in the real-time image 10, and the vessels surrounding the catheter 2 are not shown clearly, if at all, on account of the fact that the contrast medium has now broken down. The physician therefore navigates the catheter 2 with the aid of the reference image 9, which he compares with the real-time image 10. The comparison of real-time image 10 with reference image 9 for the purpose of catheter navigation has proven itself in practice. By virtue of this method, the patient need not be exposed to the contrast medium for the entire duration of an intervention that may prove to be relatively long. A brief exposure for the purpose of recording the static reference image 9 or for monitoring the progress of the examination is all that is required.

The first image recording device 1 continually supplies image signals of the vessel inner wall surrounding the catheter tip. These image signals, recorded using ultrasound, are composed of a large number of slice images oriented perpendicular to the longitudinal extent of the vessel. The image processing means 5 first generates from the image signals a planar developed view 11 of the vessel inner wall. The image processing means 5 superposes the developed view on a projection image which is displayed on the display unit 4. Optionally, the developed view can be superposed on the real-time image 10, the reference image 9 or both.

The location of the superposition of the developed view 11 in the projection image corresponds to the intravascular position of the first image recording device 1. In order to determine the location of the first image recording device 1 within the projection image, the image processing means 5 uses the position data determined by the locating device of the integrated catheter guidance and locating device 6.

The system 0 clearly displays the vessel inner wall at its anatomically correct location. The physician does not need to take his eyes off the display unit 4 and navigate the catheter 2 and inspect the blood vessel 7 at the same time.

## Claims

1. A system (0) for interventional angiography, having:
a first image recording device (1) located at a tip of a catheter for insertion into a blood vessel (7) of a patient (8), wherein the first image recording device (1) is configured to supply image signals of the vessel inner wall surrounding the catheter tip, which signals are composed of a large number of slice images oriented perpendicular to the longitudinal extend of the vessel (7), means for determining the intravascular position of the first image recording device (1), a display unit (4) for visualizing intravascular image signals recorded by means of the first image recording device (1),
a second image recording device (3) for generating a two-dimensional projection image of the blood vessel, **characterised by**:
image processing means (5) for generating from the image signals of the first image recording device (1) a planar developed view (11) of a vessel inner wall and for superposing on the two-dimensional projection image the planar developed view (11) in accordance with the intravascular position of the first image recording device (1).

2. The system as claimed in claim 1, wherein the first image recording device (1) is an intravascular ultrasound device or an optical coherence tomography device.

3. The system as claimed in either of claims 1 and 2, wherein the second image recording device (3) is a C-arm X-ray device.

4. The system as claimed in claim 1, wherein the system is configured to determine the intravascular position of the first image recording device (1) by means of a locating device.

5. A computer program for a system (0) as claimed in claim 1, wherein the computer program when run on a computer processes intravascular rendering from a first image recording device (1) inserted into a blood vessel (7) of a patient (8) and projection rendering from a second image recording device (3) and also intravascular position data from the first image recording device (1), wherein a two-dimensional development image of the inner wall of the blood vessel (7) is calculated from a number of slice images rendered by means of the first image recording device (1) and oriented perpendicular to the longitudinal extent of the blood vessel (7), and is superposed on a representation of the projection rendering a two-dimensional representation of the intravascular rendering in accordance with the intravascular position data from the first image recording device (1).

## Patentansprüche

1. System (0) zur interventionellen Angiographie, das Folgendes umfasst:
eine erste Bildaufzeichnungsvorrichtung (1) an einer Spitze eines Katheters zur Einführung in ein Blutgefäß (7) eines Patienten (8), wobei die erste Bildaufzeichnungsvorrichtung (1) konfiguriert ist, um Bildsignale der die Katheterspitze umgebenden Gefäßinnenwand zu liefern, wobei die Signale aus einer großen Anzahl von Schichtbildern bestehen, die senkrecht zu dem Längsverlauf des Gefäßes (7) ausgerichtet sind,
Mittel zum Bestimmen der intravaskulären Position der ersten Bildaufzeichnungsvorrichtung (1),
eine Anzeigeeinheit (4) zum Visualisieren der mit Hilfe der ersten Bildaufzeichnungsvorrichtung (1) aufgezeichneten intravaskulären Bildsignale,
eine zweite Bildaufzeichnungsvorrichtung (3) zum Erzeugen eines zweidimensionalen Projektionsbildes des Blutgefäßes, **gekennzeichnet durch**: Bildverarbeitungsmittel (5), um anhand der Bildsignale der ersten Bildaufzeichnungsvorrichtung (1) eine planare entwickelte Ansicht (11) einer Gefäßinnenwand zu erzeugen und um dem zweidimensionalen Projektionsbild die planare entwickelte Ansicht (11) entsprechend der intravaskulären Position der ersten Bildaufzeichnungsvorrichtung (1) zu überlagern.

2. System nach Anspruch 1, wobei die erste Bildaufzeichnungsvorrichtung (1) eine intravaskuläre Ultraschallvorrichtung oder eine Vorrichtung für die optische Kohärenztomographie ist.

3. System nach einem der Ansprüche 1 und 2, wobei die zweite Bildaufzeichnungsvorrichtung (3) eine C-Bogen-Röntgenvorrichtung ist.

4. System nach Anspruch 1, wobei das System konfiguriert ist, um die intravaskuläre Position der ersten Bildaufzeichnungsvorrichtung (1) mit Hilfe einer Lokalisierungsvorrichtung zu bestimmen.

5. Computerprogramm für ein System (0) nach Anspruch 1, wobei das Computerprogramm, wenn es auf einem Computer ausgeführt wird, die intravaskuläre Wiedergabe von einer ersten Bildaufzeichnungsvorrichtung (1), die in ein Blutgefäß (7) eines Patienten (8) eingeführt ist, und die Projektionswiedergabe von einer zweiten Bildaufzeichnungsvorrichtung (3) und auch intravaskuläre Positionsdaten von der ersten Bildaufzeichnungsvorrichtung (1) verarbeitet, wobei ein zweidimensionales Entwicklungsbild der Innenwand des Blutgefäßes (7) anhand einer Anzahl von Schichtbildern berechnet wird, die mit Hilfe der ersten Bildaufzeichnungsvorrichtung (1) wiedergegeben werden und senkrecht zum Längsverlauf des Blutgefäßes (7) ausgerichtet sind, und wobei der Darstellung der Projektionswiedergabe eine zweidimensionale Darstellung der intravaskulären Wiedergabe entsprechend der intravaskulären Positionsdaten von der ersten Bildaufzeichnungsvorrichtung (1) überlagert wird.

## Revendications

1. Système (0) pour l'angiographie interventionnelle ayant :
un premier dispositif d'enregistrement d'image (1) qui se situe à une extrémité d'un cathéter qui est à insérer dans un vaisseau sanguin (7) d'un patient (8), dans lequel le premier dispositif d'enregistrement d'image (1) est configuré de manière à fournir des signaux d'image de la paroi intérieure du vaisseau qui entoure l'extrémité du cathéter, lesquels signaux sont composés d'un grand nombre d'images de tranche qui sont orientées perpendiculairement au prolongement longitudinal du vaisseau (7), des moyens pour déterminer la position intra-vasculaire du premier dispositif d'enregistrement d'image (1), une unité d'affichage (4) pour visualiser des signaux d'image intra-vasculaire qui sont enregistrés au moyen du premier dispositif d'enregistrement d'image (1),
un deuxième dispositif d'enregistrement d'image (3) pour générer une image de projection bidimensionnelle du vaisseau sanguin, **caractérisé par** :
des moyens de traitement d'image (5) pour générer à partir des signaux d'image du premier dispositif d'enregistrement d'image (1) une vue développée planaire (11) d'une paroi intérieure du vaisseau de manière à superposer sur l'image de projection bidimensionnelle la vue développée planaire (11) en conformité avec la position intra-vasculaire du premier dispositif d'enregistrement d'image (1).

2. Système selon la revendication 1, dans lequel le premier dispositif d'enregistrement d'image (1) est un dispositif intra-vasculaire à ultrasons ou un dispositif optique de tomographie de cohérence.

3. System selon l'une quelconque des revendications précédentes 1 à 2, dans lequel le deuxième dispositif d'enregistrement d'image (3) est un dispositif à rayons X à bras en C.

4. Système selon la revendication 1, dans lequel le système est configuré de manière à déterminer la position intra-vasculaire du premier dispositif d'enregistrement d'image (1) au moyen d'un dispositif de localisation.

5. Programme informatique pour un système (0) selon la revendication 1, dans lequel le programme informatique, lorsqu'il est exécuté sur un ordinateur, traite le rendu intra-vasculaire à partir d'un premier dispositif d'enregistrement d'image (1) qui est inséré dans un vaisseau sanguin (7) d'un patient (8) et le rendu de projection à partir d'un deuxième dispositif d'enregistrement d'image (3) et également les données de la position intra-vasculaire à partir du premier dispositif d'enregistrement d'image (1), dans lequel une image bidimensionnelle de développement de la paroi intérieure du vaisseau sanguin (7) est calculée à partir d'un certain nombre d'images de tranche qui sont rendues au moyen du premier dispositif d'enregistrement d'image (1) et qui sont orientées perpendiculairement au prolongement longitudinal du vaisseau sanguin (7), et est superposée sur une représentation de la projection qui rend une représentation bidimensionnelle du rendu intra-vasculaire en conformité avec les données de la position intra-vasculaire à partir du premier dispositif d'enregistrement d'image (1).
